# EUROPÄISCHE PATENTSCHRIFT

(11) **EP 2 912 529 B1**
(45) Veröffentlichungstag und Bekanntmachung des Hinweises auf die Patenterteilung: **03.05.2017**
(21) Anmeldenummer: 14741866.9
(22) Anmeldetag: 18.07.2014
(51) Int. Cl.: G06C 1/00, A61P 7/12, A61M 5/315

(54) **VORRICHTUNG ZUR ERMITTLUNG EINER ZU SPRITZENDEN INSULINMENGE BEI DIABETES-PATIENTEN**
DEVICE FOR THE DETERMINATION OF AN AMOUNT OF INSULINE FOR DIABETES-PATIENTS TO BE INJECTED
APPAREIL À LA DÉTERMINATION DE LA QUANTITÉ D'INSULINE À INJECTER AUX PATIENTS DIABÉTIQUES

(30) Priorität: 17.01.2014 DE 102014200866
(43) Veröffentlichungstag der Anmeldung: 02.09.2015
(73) Patentinhaber: IWA-F. RIEHLE GMBH & CO KG, 73770 Denkendorf (DE)
(72) Erfinder: RIEHLE, Harald, 70178 Stuttgart (DE); GREBING, Gerhard, 72622 Nuertingen (DE)
(74) Vertreter: DREISS Patentanwälte PartG mbB
(86) Internationale Anmeldenummer: PCT/EP2014/065555
(87) Internationale Veröffentlichungsnummer: WO 2015/106839

(56) Entgegenhaltungen:
- WO-A1-2005/091208
- WO-A2-2006/079124
- DE-A1- 2 721 302
- GB-A- 2 189 633
- US-A- 4 313 054
- US-B1- 6 543 682

## Beschreibung

Die Erfindung betrifft eine Vorrichtung zur Ermittlung einer zu spritzenden Insulinmenge bei Diabetes-Patienten.

Diabetespatienten müssen regelmäßig in Abhängigkeit der Anzahl von aufzunehmenden Broteinheiten bzw. Kohlehydrateinheiten häufig mehrmals täglich, insbesondere morgens, mittags, abends und spätabends, Insulinmengen spritzen. Die Menge des zu spritzenden Insulins, d. h. die Anzahl der zu spritzenden Insulineinheiten, hängt aber nicht nur von der Menge der aufgenommenen oder unmittelbar aufzunehmenden Broteinheiten oder Kohlehydrateinheiten ab, sondern auch von tageszeitspezifischen Parametern, die zudem patientenspezifisch zu ermitteln sind. Man identifiziert diesbezüglich den sogenannten Insulinfaktor, der typischerweise für die jeweilige Tageszeit, also morgens, mittags, abends sowie spätabends, vom behandelnden Arzt oder Therapeuten für einen Patienten ermittelt und gewissermaßen festgeschrieben wird, und den Blutzucker-Zielwert sowie den Blutzucker-Senkungswert, die ebenfalls patientenspezifisch für die jeweilige Tageszeit ermittelt und für den Patienten zugrunde gelegt werden. Des Weiteren geht die Ermittlung einer zu spritzenden Insulinmenge stets einher mit der Messung des aktuellen Blutzuckerwerts. Dabei wird eine Abweichung zwischen dem aktuellen Blutzuckerwert und dem vorerwähnen Blutzucker-Zielwert ermittelt und mit dem vorstehenden Blutzucker-Senkungswert verknüpft, um hieraus einen Korrekturwert bzw. eine Korrekturmenge an Insulin zu ermitteln, die unabhängig von der Menge der aufzunehmenden Broteinheiten oder Kohlehydrateinheiten zusätzlich zu spritzen ist.

WO 2005/081173 A1 und WO 2006/079124 A2 offenbaren Schiebervorrichtungen mit mehreren in einer Längsrichtung und parallel zueinander verschiebbaren Schieberzungen, die dazu dienen sollen, in Abhängigkeit von Parametern, wie aktuell gemessener Blutzucker, aufgenommene oder aufzunehmende Nahrung, eine zu spritzende Insulinmenge zu errechnen. In der WO 2006/079124 A2 ist neben einer Schiebervorrichtung zusätzlich eine Drehscheibenvorrichtung mit drei gegeneinander verdrehbaren Drehscheiben offenbart, die demselben Ziel dient. Mit DE 2721302 A1 wurde bereits der Vorschlag unterbreitet, bei einer Drehscheibenvorrichtung zwei gegeneinander verdrehbare Scheiben mittels einer dazwischen angeordneten Haftfolie gegeneinander lösbar zu fixieren. US 3,514,582 zeigt einen Rechenschieber mit einer längs verschiebbaren Schieberzunge und einer mit dem Daumen quer verschiebbaren Ablesemarkierung. US 6,543,682 B1 offenbart eine Drehscheibenvorrichtung zur Ermittlung einer zu spritzenden Insulinmenge, aus geschichtet angeordneten Flachmaterialabschnitten, die einen scheibenförmigen Gehäusekörper bilden, mit mehreren Drehscheiben, die um eine gemeinsame Drehachse, die orthogonal zu den Flachmaterialabschnitten verläuft, zueinander drehbar sind. US 4,313,054 offenbart eine Rechenvorrichtung mit einer ersten Drehscheibe, mehreren Eingabeskalen mit jeweils äquidistanter Strichteilung, einer Ergebnisskala, wobei die mehreren Eingabeskalen in verschiedenen radialen Abständen und jeweils konzentrisch zur Drehachse angeordnet sind , mit Einstellmarken, Ablesewertskalen mit jeweils äquidistanter Strichteilung, wobei jeder Ablesewertskala ein zweiter Parameter zugeordnet ist, und dann bei einem Wert der Ablesewertskala als Ablesemarke ein Wert auf der Ergebnisskala ablesbar ist. Ferner sind Fenster zur Einsichtnahme vorgesehen.

Der vorliegenden Erfindung liegt die Aufgabe zugrunde, eine einfach und vor allem sicher zu handhabende Vorrichtung zur Ermittlung einer zu spritzenden Insulinmenge bereitzustellen, die insbesondere im Hinblick auf an Diabetes I und II erkrankte Kinder, aber auch Erwachsene, einen sicheren Schutz vor Fehlbedienungen zu bieten geeignet ist.

Diese Aufgabe wird nach der Erfindung durch eine Drehscheibenvorrichtung mit den Merkmalen des Anspruchs 1 gelöst.

Nicht beansprucht ist eine Schiebervorrichtung zur Ermittlung einer zu spritzenden Insulinmenge, aus geschichtet angeordneten Flachmaterialabschnitten, die einen scheibenförmigen Gehäusekörper bilden, mit mehreren gegeneinander verschiebbaren Schieberzungen, wobei eine erste Schieberzunge mehrere Eingabeskalen mit jeweils äquidistanter Strichteilung und eine Ergebnisskala aufweist und für den bestimmungsgemäßen Gebrauch manuell ergreifbar und in einer ersten Richtung verschiebbar ist,
wobei jeder Eingabeskala ein erster Parameter zugeordnet ist,
wobei eine zweite Schieberzunge in einer zweiten Richtung, die quer zur ersten Richtung verläuft, verschiebbar ist und ein Fenster mit einer Einstellmarke aufweist, welches bezüglich des Gehäusekörpers derart positionierbar ist, dass eine visuelle Einsichtnahme wahlweise auf jede der Eingabeskalen ermöglicht ist,
wobei neben der zweiten Schieberzunge eine dritte Schieberzunge in der zweiten Richtung verschiebbar ist und eine Mehrzahl von Fenstern aufweist, die derart ausgebildet und angeordnet sind, dass ein jeweiliges Fenster bei einer entsprechend wählbaren Verschiebestellung der dritten Schieberzunge bezüglich des Gehäusekörpers eine visuelle Einsichtnahme auf die Ergebnisskala ermöglicht, und
wobei jedes Fenster der dritten Schieberzunge randseitig entlang der ersten Richtung eine Ablesewertskala mit jeweils äquidistanter Strichteilung aufweist, wobei jeder Ablesewertskala ein zweiter Parameter zugeordnet ist, und
wobei nach Positionierung der zweiten und dritten Schieberzunge in Bezug auf den Gehäusekörper entsprechend dem Wert des ersten und zweiten Parameters die erste Schieberzunge gegenüber der Einstellmarke am Fenster der zweiten Schieberzunge positionierbar ist und dann bei einem Wert der Ablesewertskala als Ablesemarke ein Wert auf der Ergebnisskala ablesbar ist, der der zu spritzenden Insulinmenge entspricht,
und wobei die zweite Schieberzunge und die dritte Schieberzunge relativ zu dem Gehäusekörper in einer jeweiligen Positionierung unverschiebbar stellbar sind.

Bei dieser Schiebervorrichtung werden die mehreren Schieberzungen bzw. deren verschiedene Skalen derart relativ zueinander positioniert, dass ein Benutzer bei einem spezifischen Wert der Ablesewertskala als Ablesemarke dann unmittelbar direkt die zu spritzende Insulinmenge ablesen kann, wobei dieser spezifische Wert der Ablesewertskala dem gerade gemessenen aktuellen Blutzuckerwert entspricht. Die verschiedenen Eingabeskalen und deren jeweilige äquidistante Strichteilung entsprechen der Eingabe der Menge der aufzunehmenden Broteinheiten bzw. Kohlehydrateinheiten unter Zugrundelegung eines spezifischen Parameters insbesondere in Form des vorerwähnten Insulinfaktors für eine jeweilige Eingabeskala. Durch Positionierung der zweiten Schieberzunge mit ihrem Fenster über einer spezifischen Eingabeskala wird der zugrunde zu legende erste Parameter für die auszuführende Berechnung festgelegt. Durch Positionierung der dritten Schieberzunge derart, dass eines ihrer Fenster eine visuelle Einsichtnahme auf die Ergebnisskala der ersten Schieberzunge ermöglicht, wird eine dem zweiten Parameter entsprechende spezifische Ablesewertskala bei der Ergebnisskala der ersten Schieberzunge positioniert und auf diese Weise der zweite Parameter für die vorzunehmende Berechnung festgelegt. Es braucht nun lediglich die erste Schieberzunge in eine solche Verschiebestellung gebracht zu werden, dass die Einstellmarke am Fenster der zweiten Schieberzunge bei der Menge der aufzunehmenden Broteinheiten bzw. Kohlehydrateinheiten bei der Eingabeskala positioniert wird. Nach einer Messung des aktuellen Blutzuckerwerts kann dann die zu spritzende Insulinmenge im Fenster der dritten Schieberzunge auf der Ergebnisskala unmittelbar abgelesen werden, und zwar bei dem aktuell gemessenen Blutzuckerwert der Ablesewertskala, der hierfür gewissermaßen eine Ablesemarke bildet.

Da der erste Parameter und der zweite Parameter patientenspezifisch und für eine jeweilige Tageszeit vom Arzt oder Therapeuten vorgeschrieben werden, wäre es sinnvoll, dass der Diabetes Patient für jede Tageszeit eine gesonderte Vorrichtung vorhält, bei der die zweite und dritte Schieberzunge in einer der Tageszeit entsprechenden Schiebeposition bereits eingestellt sind. Insoweit erweist es sich als vorteilhaft, wenn eine Verschiebung der zweiten Schieberzunge oder der dritten Schieberzunge und vorzugsweise dieser beiden Schieberzungen nicht werkzeuglos ausführbar ist oder nur nach Ablösen eines Sicherungselements oder nach Freigeben einer Zugangsöffnung in das Innere des Gehäusekörpers möglich ist. Hierdurch kann ein Schutz gegen unbeabsichtigtes Verstellen der Schiebestellung der zweiten und dritten Schieberzunge erreicht werden.

Bei der erfindungsgemäßen Drehscheibenvorrichtung werden anstelle von verschiebbaren Schieberzungen Flachmaterialabschnitte in Form von Drehscheiben verwendet. Dabei weist eine erste Drehscheibe mehrere Eingabeskalen und eine Ergebnisskala auf. Die mehreren Eingabeskalen sind in radialer Richtung aufeinander folgend angeordnet und jeweils in Umfangsrichtung, also konzentrisch zur Drehachse, erstreckt.

Eine zweite Drehscheibe umfasst eine Mehrzahl von Fenstern, die in verschiedenen radialen Abständen angeordnet und in Umfangsrichtung, also konzentrisch, erstreckt und vorzugsweise in Umfangsrichtung zueinander versetzt angeordnet sind. Durch Drehung der zweiten Drehscheibe gegenüber der ersten Drehscheibe kann somit eine spezifische Eingabeskala in den Bereich des Sichtfensters der zweiten Drehscheibe gebracht werden. Des Weiteren ist eine dritte Drehscheibe mit einer Mehrzahl von konzentrisch und in Umfangsrichtung aufeinander folgend angeordneten Ablesewertskalen vorgesehen, die durch Drehung eine spezifische Ablesewertskala der dritten Drehscheibe in Ableseposition zur Ergebnisskala auf der ersten Drehscheibe bringen kann. Die Reihenfolge der Anordnung der zweiten Drehscheibe und der dritten Drehscheibe kann auch umgekehrt sein und ist hier rein beispielhaft. Wie bei der Schiebervorrichtung sind bei den spezifischen Abständen der Strichteilung der jeweiligen Skalen der zugrunde gelegte Algorithmus und der zugrunde gelegte patientenspezifische und tageszeitspezifische Parameter hinterlegt. Für die Benutzung der Drehscheibenvorrichtung wird dabei die zweite Drehscheibe und die dritte Drehscheibe in eine den zugrunde zu legenden Parametern entsprechende relative Position zueinander verdreht und in dieser Position vorzugsweise gegeneinander fixiert, was nachfolgend noch im Einzelnen erläutert werden wird. Es wird dann eine Drehstellung der zweiten und der dritten Drehscheibe relativ zur mittels der zweiten Drehscheibe ausgewählten Eingabeskala der ersten Drehscheibe vorgenommen, so dass die aufzunehmende Menge an Broteinheiten bzw. Kohlehydrateinheiten über die Einstellmarke beim Fenster der zweiten Drehscheibe eingestellt wird. Es kann dann wie bei der Schiebervorrichtung die zu spritzende Insulinmenge unmittelbar bei dem gemessenen aktuellen Blutzuckerwert der Ablesewertskala als Ablesemarke auf der Ergebnisskala abgelesen werden.

Die Vorteile der erfindungsgemäßen Drehscheibenvorrichtung entsprechen den vorausgehend im Zusammenhang mit der erfindungsgemäßen Schiebervorrichtung erläuterten Vorteilen. Ihre Handhabung dürfte jedoch noch einfacher und benutzerfreundlicher sein, was auch ihre Akzeptanz beim Benutzer erhöhen dürfte.

In weiterer erfindungsgemäßer Ausbildung der Drehscheibenvorrichtung erweist es sich als vorteilhaft, wenn eine vierte Drehscheibe als Deckscheibe vorgesehen ist, die ein radial und in Umfangsrichtung erstrecktes Fenster aufweist, welches in einer wählbaren Drehstellung eine Einsichtnahme auf die Ablesewertskala der dritten Drehscheibe und auf die Ergebnisskala der ersten Drehscheibe und durch ein Fenster der zweiten Drehscheibe hindurch auf die Eingabeskala der ersten Drehscheibe ermöglicht. Auf diese Weise erhält der Benutzer spezifische Einsicht auf die entsprechend der Positionierung der zweiten und dritten Drehscheibe zueinander ausgewählten benutzerrelevanten Skalen. Weiter erweist es sich als vorteilhaft, wenn die zweite und die dritte Drehscheibe und gegebenenfalls die vierte Drehscheibe in einer gewählten Drehstellung zueinander unverdrehbar stellbar sind. Auf diese Weise lassen sich die vom Arzt oder Therapeuten vorgegebenen patientenspezifischen und tageszeitspezifischen Parameter einstellen und für den weiteren Gebrauch fixieren.

Zum Unverdrehbarstellen der zweiten und der dritten Drehscheibe gibt es eine Vielzahl von Möglichkeiten, die sich in der Handhabung, der technischen Realisierbarkeit und der Benutzerakzeptanz unterscheiden können. Beispielsweise wird vorgeschlagen, dass zum Unverdrehbarstellen der zweiten und der dritten Drehscheibe und gegebenenfalls der vierten Drehscheibe eine insbesondere lösbare Klebeverbindung oder ein die Scheiben klemmschlüssig oder formschlüssig miteinander koppelndes mechanisches Element, insbesondere in Form einer Nietverbindung oder Schraubverbindung, vorgesehen ist.

Da Kopplungselemente zwischen der zweiten und der dritten Drehscheibe in axialer Richtung aufragen, erweist es sich als vorteilhaft, dass eine Abstandhalterscheibe zwischen der ersten und der zweiten Drehscheibe vorgesehen ist und radial außerhalb der Abstandhalterscheibe die insbesondere lösbare Klebeverbindung oder ein die Scheiben klemmschlüssig oder formschlüssig miteinander koppelndes mechanisches Element, insbesondere in Form einer Nietverbindung oder Schraubverbindung oder Verplombung, vorgesehen ist. Auf diese Weise kann eine störende Unebenheit vermieden werden, und insbesondere wird Abrieb oder sonstige Behinderung bei der Benutzung der Drehscheibenvorrichtung vermieden oder zumindest reduziert.

Wenn ein mechanisches Element zum Verbinden der Drehscheiben verwendet wird, so kann es sich als vorteilhaft erweisen, wenn das die zweite, dritte und vierte Drehscheibe formschlüssig miteinander koppelnde mechanische Element eine Plombe aus zwei ineinandergreifenden und durch Lochausstanzungen in der zweiten, dritten und vierten Drehscheibe hindurchgreifenden Kunststoffspritzgussteilen ist, wobei die Plombe eine Sollbruchstelle aufweist, an der sie vom Arzt bei einer beabsichtigten Neueinstellung der drei Drehscheiben aufgebrochen und durch eine neue Plombe ersetzt werden kann.

Weiter erweist es sich als vorteilhaft hinsichtlich der Handhabbarkeit der Vorrichtung, wenn die erste Drehscheibe über die übrigen Drehscheiben radial vorsteht und dort eine Haltezone zum Ergreifen und Halten der Vorrichtung während der Bedienung bildet. Auch beim Verstellen der zweiten und dritten Scheibe relativ zueinander und beim Unverdrehbarstellen einer gewählten Position kann sich diese Ausbildung der ersten Drehscheibe mit einer Haltezone als besonders vorteilhaft erweisen.

Es erweist sich auch als vorteilhaft, wenn die Parameterwerte in einer Umfangsrichtung aufeinanderfolgend visuell wahrnehmbar vorgesehen sind und in einer gewählten Drehstellung der zweiten und der dritten Drehscheibe zueinander der erste und der zweite Parameterwert auf der Sichtseite der Vorrichtung visuell wahrnehmbar sind.

Sofern zum Unverdrehbarstellen der zweiten, dritten und vierten Drehscheibe eine Klebeverbindung eingesetzt wird, so erweist es sich als besonders vorteilhaft, wenn ein Klebeetikett vorgesehen ist, welches auf die Sichtseite der vierten Drehscheibe aufbringbar und dabei durch eine Ausnehmung in der vierten Drehscheibe hindurch auf der dritten Drehscheibe anhaftbar und durch eine Ausnehmung in der dritten Drehscheibe hindurch auf der zweiten Drehscheibe anhaftbar ist, so dass die zweite, dritte und vierte Drehscheibe unverdrehbar zueinander gestellt sind. Bei dieser Ausführungsform wird davon ausgegangen, dass die erste bis vierte Drehscheibe in dieser Reihenfolge 1-2-3-4 aufeinanderfolgend angeordnet sind.

Wenn die Reihenfolge der zweiten und dritten Drehscheibe umgekehrt ist, also 1-3-2-4, so erweist es sich als vorteilhaft, wenn zum Unverdrehbarstellen der zweiten, dritten und vierten Drehscheibe ein Klebeetikett vorgesehen ist, welches auf die Sichtseite der vierten Drehscheibe aufbringbar und dabei durch eine Ausnehmung in der vierten Drehscheibe hindurch auf der zweiten Drehscheibe anhaftbar und durch eine Ausnehmung in der zweiten Drehscheibe hindurch auf der dritten Drehscheibe anhaftbar ist, so dass die zweite, dritte und vierte Drehscheibe unverdrehbar zueinander gestellt sind.

Bei beiden Ausführungsformen wird jedenfalls in der vierten Drehscheibe, also der Deckscheibe, und in der daran angrenzenden Drehscheibe, also in der dritten oder zweiten Drehscheibe, eine Ausnehmung vorgesehen, und alle drei Drehscheiben werden durch das auf die Deckscheibe oben flächenhaft aufzubringende Klebeetikett miteinander und gegeneinander fixiert und damit unverdrehbar gestellt. Es erweist sich als besonders vorteilhaft, wenn dieses Klebeetikett zugleich als Informationsträger fungiert und der behandelnde Arzt oder Therapeut die vorgenommene Einstellung der Parameterwerte durch Fixieren der drei Scheiben zueinander auf dem Etikett dokumentieren kann.

Weiter erweist es sich als vorteilhaft, wenn das Klebeetikett so ausgebildet ist, dass es nicht zerstörungsfrei ablösbar ist. Hierfür könnte das Klebeetikett Sollbruchlinien aufweisen, so dass es beim Abziehen teilweise unter Selbstzerstörung an der Drehscheibenvorrichtung verbleibt.

Es erweist sich insbesondere als vorteilhaft, wenn die Ausnehmung in der zweiten oder dritten Drehscheibe langlochförmig, nierenförmig oder in Form einer Reihe von in Umfangs- oder Drehrichtung aufeinanderfolgend angeordneten Ausnehmungen ausgebildet ist, wobei die Abmessung der Ausnehmung oder der Ausnehmungen in der zweiten oder dritten Drehscheibe kleiner ist als die Abmessung der Ausnehmung in der vierten Drehscheibe, so dass das Klebeetikett auf einem Umgebungsbereich der Ausnehmung oder der Ausnehmungen in der zweiten oder dritten Drehscheibe anhaftbar ist. Auf diese Weise kann sichergestellt werden, dass durch einfaches flächenhaftes Aufbringen eines Klebeetiketts auf die vierte Drehscheibe alle drei Scheiben miteinander fixiert und hierdurch unverdrehbar gestellt werden.

Die mehreren Eingabeskalen der Schiebervorrichtung bzw. der Drehscheibenvorrichtung haben zwar jeweils vorzugsweise äquidistante Strichteilung, sie unterscheiden sich jedoch in den Abständen der jeweiligen Strichteilung voneinander. Bei der Eingabeskala entspricht das Verhältnis der Abstände der Strichteilung der jeweiligen Eingabeskalen zueinander dem Verhältnis der einer jeweiligen Eingabeskala zugeordneten ersten Parameter. Da bei der Ablesewertskala eine Division ausgeführt wird, entspricht das Verhältnis der Abstände der Strichteilung der jeweiligen Ablesewertskalen zueinander dem umgekehrten Verhältnis des einer jeweiligen Ablesewertskala zugeordneten zweiten Parameters.

Wie eingangs erwähnt wäre es sinnvoll, dass der Diabetes -Patient für jede Tageszeit eine voreingestellte Schieber- oder Drehscheibenvorrichtung vorhält. Bei der Drehscheibenvorrichtung wäre es auch denkbar, dass die erste Drehscheibe als Grundscheibe vorgehalten wird und dass daran für jede Tageszeit voreingestellte Verbunde aus den übrigen Drehscheiben drehbar befestigt werden. Hierfür könnte die Drehverbindung bei der Drehachse lösbar ausgebildet werden, etwa durch eine Druckknopfverbindung, die einfach einrastbar und lösbar ist.

Von der vorausgehend geschilderten Erfindung sind auch Schiebervorrichtungen und Drehscheibenvorrichtungen erfasst, die zu anderen Zwecken als zur Ermittlung einer zu spritzenden Insulinmenge verwendbar sind. Bei solchen anderen Zwecken könnten auch andere Algorithmen durch spezifische Ausgestaltung der jeweiligen Skalen und deren Strichteilungen abgebildet bzw. umgesetzt werden.

Weitere Merkmale, Einzelheiten und Vorteile der Erfindung ergeben sich aus den beigefügten Patentansprüchen, der zeichnerischen Darstellung und nachfolgenden Beschreibung bevorzugter Ausführungsformen der Erfindung.

In der Zeichnung zeigt:
Figuren 1a-d vier Ebenen einer nicht beanspruchten Schiebervorrichtung;
Figur 2 eine Draufsicht auf die Schiebervorrichtung;
Figuren 3a-e verschiedene Ebenen einer erfindungsgemäßen Drehscheibenvorrichtung;
Figur 4 eine Draufsicht auf die Drehscheibenvorrichtung und
Figuren 5a,b je ein Ausführungsbeispiel für ein Element zum Fixieren der Drehstellung von Drehscheiben gegeneinander;
Figuren 6a - e, eine weitere Ausführungsform der erfindungsgemäßen Drehscheibenvorrichtung, bei der drei Drehscheiben mittels eines Klebeetiketts unverstellbar zueinander gestellt werden können.
Figur 7 eine Tabelle.

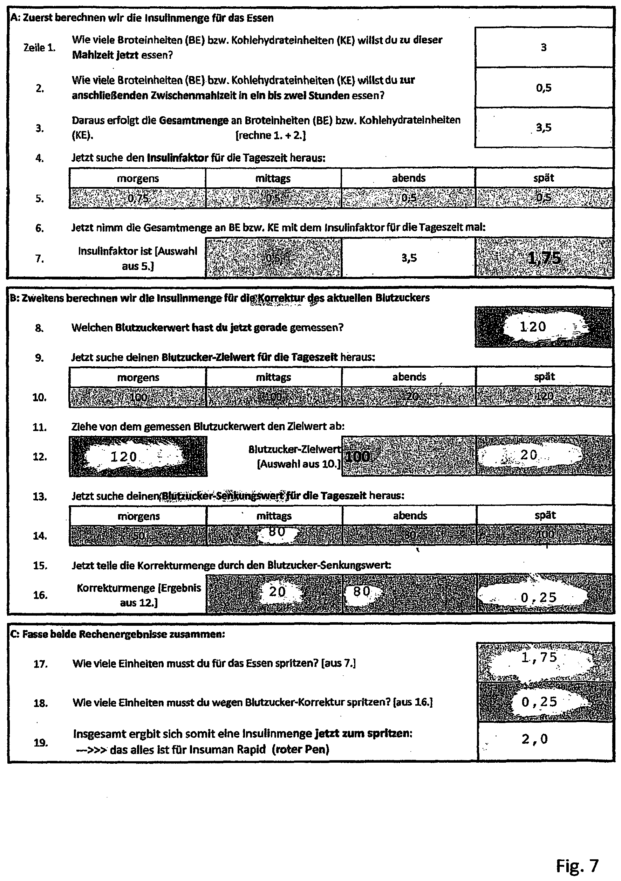

Zunächst wird anhand der Tabelle gemäß Figur 7 eine sich typischerweise stellende Berechnung der zu spritzenden Insulinmenge anhand beispielhafter Broteinheiten bzw. Kohlehydrateinheiten und anhand beispielhafter Parameter für den Insulinfaktor und den Blutzucker-Senkungswert und anhand eines beispielhaft gemessenen Blutzuckerwerts erörtert.

Tabelle: Berechnung der Insulinmenge gemäß Figur 7:
Es sind in den Zeilen 1 bis 3 der Tabelle insgesamt 3,5 Broteinheiten bzw. Kohlehydrateinheiten zugrunde gelegt, die zu anstehenden Mahlzeiten gegessen werden sollen. In der Zeile 5 sind für verschiedene Tageszeiten, nämlich morgens, mittags, abends sowie spätabends zugrunde gelegte patientenspezifische Insulinfaktoren gezeigt, wobei beispielhaft für mittags in der Zeile 7 der Parameter 0,5 gewählt ist. Aus diesem Parameter als Insulinfaktor ergeben sich multipliziert mit den 3,5 Broteinheiten bzw. Kohlehydrateinheiten rechnerisch 1,75 Insulineinheiten (Zeile 7). In der Zeile 8 wird ein aktuell gemessener Blutzuckerwert von 120 zugrunde gelegt. In der Zeile 10 sind für die Tageszeiten die tageszeitspezifischen Blutzucker-Zielwerte eingetragen. In der Zeile 12 wird die Abweichung des aktuellen Blutzuckerwerts von 120 von dem für mittags zugrunde zu legenden Blutzucker-Zielwert von 100 angegeben, nämlich eine Differenz von 20. In der Zeile 14 sind die patientenspezifischen und tageszeitspezifischen Blutzucker-Senkungswerte eingetragen. In der Zeile 16 wird der Differenzwert von 20 aus der Zeile 12 durch den tageszeitspezifischen Blutzucker-Senkungswert von mittags 80 dividiert, wobei sich 0,25 Insulineinheiten ergeben. In der Zeile 19 werden die in der Zeile 7 ermittelten Insulineinheiten von 1,75 mit der Insulinkorrekturmenge aus Zeile 16 addiert, wobei sich 2,0 ergibt. Dies ist derjenige Wert, den der Patient im Zusammenhang mit der anstehenden Mittagsmahlzeit und der nachfolgenden Zwischenmahlzeit spritzen muss.

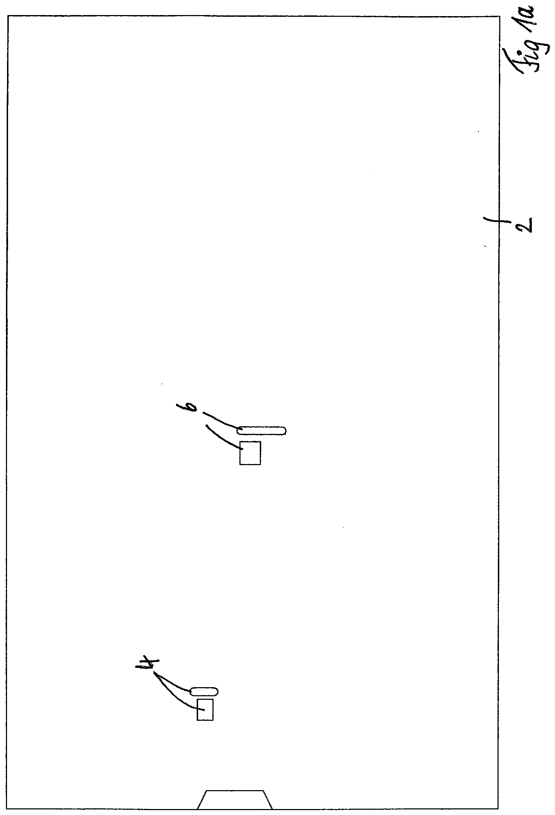

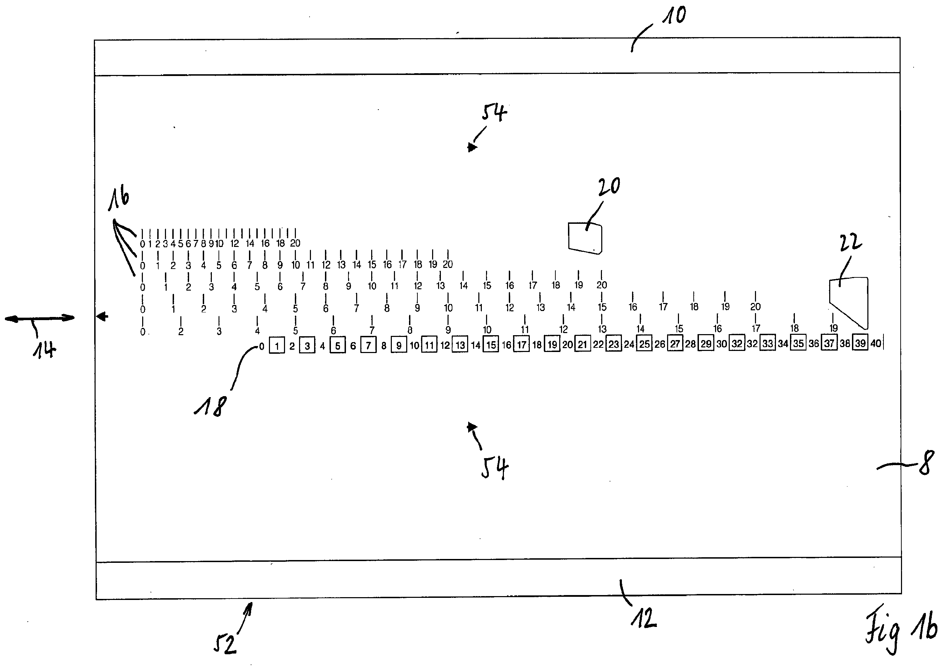

Figuren 1a bis d zeigen vier Ebenen einer erfindungsgemäßen Schiebervorrichtung 1 (im zerlegten Zustand), und zwar von unten nach oben, also in Richtung auf die dem Benutzer im Betrieb zugewandte Sichtseite der Schiebervorrichtung. Die Ansicht dieser Ebenen ist jeweils ausgehend vom Betrachter, also in Richtung auf die dem Betrachter zugewandte Seite der betreffenden Ebene dargestellt. Figur 1a zeigt eine rückseitige Deckschicht 2 mit noch zu erläuternden Fenstern 4 und 6 in Form von durchgehenden Ausnehmungen. Figur 1b zeigt eine zweite Ebene in Form einer ersten Schieberzunge 8 und zwei feststehenden langgestreckten Flachmaterialabschnitten 10 und 12 jeweils randseitig oben und unten, welche zum bestimmungsgemäßen Gebrauch unlösbar mit der rückwärtigen Deckschicht 2 verklebt sind. Sie bilden eine lineare Führung für die in Richtung des Doppelpfeils 14 hin und her verschiebbare Schieberzunge 8. Die erste Schieberzunge weist fünf Eingabeskalen 16 und eine Ergebnisskala 18 auf, wobei die Skalen in der Verschieberichtung 14 erstreckt sind und quer zur Verschieberichtung 14 nebeneinander angeordnet sind. In der ersten Schieberzunge 8 sind weiterhin zwei noch zu erläuternde Fenster 20 und 22 in Form von Durchgriffsausnehmungen ausgebildet.

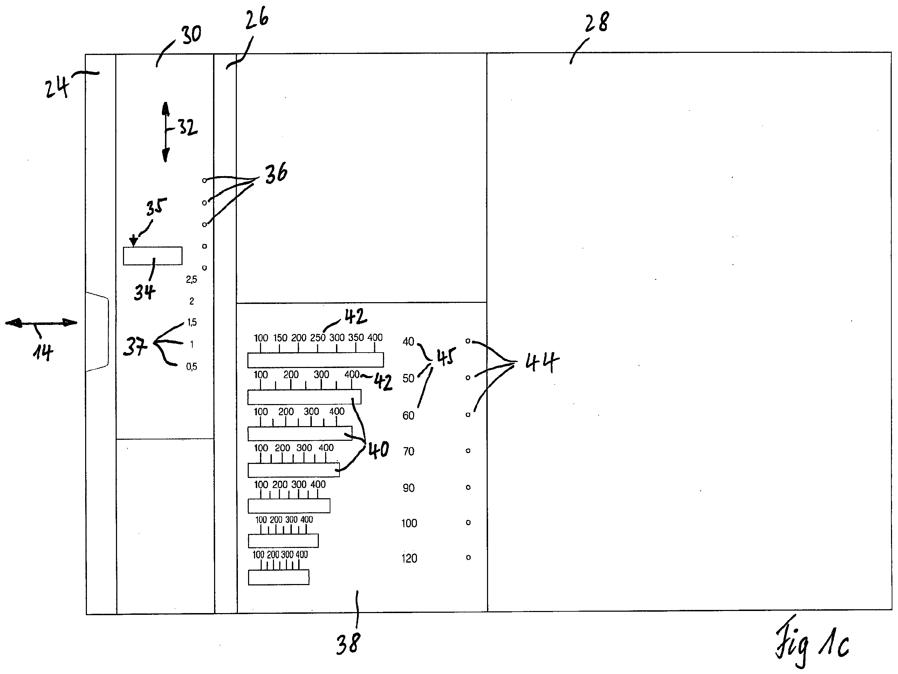

Figur 1c zeigt eine dritte Ebene. Man erkennt quer zur Verschieberichtung 14 erstreckte streifenförmige Flachmaterialabschnitte 24, 26, welche an ihrem oberen und unteren Ende jeweils mit den streifenförmigen Flachmaterialabschnitten 10 und 12 der zweiten Ebene unlösbar verbunden, insbesondere verklebt sind. Des Weiteren ist ein ausladender Flachmaterialabschnitt 28 ersichtlich, der ebenfalls oben und unten mit den streifenförmigen Flachmaterialabschnitten 10, 12 unlösbar verbunden ist. Zwischen den streifenförmigen Flachmaterialabschnitten 24 und 26 ist eine zweite Schieberzunge 30 orthogonal zur Verschieberichtung 14 der ersten Schieberzunge in einer Richtung 32 verschiebbar vorgesehen. Sie weist ein Fenster 34 mit einer Einstellmarke 35 auf, welches als Durchgriffsausnehmung oder als visuell transparenter Bereich ausgebildet ist. Durch Verschieben der zweiten Schieberzunge 30 in ihrer Verschieberichtung 32 lässt sich das Fenster 34 in der Richtung 32 gegenüber der darunter befindlichen ersten Schieberzunge an verschiedenen Stellen positionieren, so dass wahlweise eine der Eingabeskalen 16 durch das Fenster 34 hindurch visuell wahrnehmbar ist. Zum Verstellen der zweiten Schieberzunge 30 ist ein in der Verschieberichtung 32 erstrecktes Lochraster 36 vorgesehen, dessen Benutzung sogleich beschrieben werden wird.

Zwischen dem streifenförmigen Flachmaterialabschnitt 26 und dem ausladenden Flachmaterialabschnitt 28 ist eine dritte Schieberzunge 38 vorgesehen, die ebenfalls in der Verschieberichtung 32, also orthogonal zur Verschieberichtung 14 der ersten Schieberzunge 8, verschiebbar ist. Die dritte Schieberzunge 38 umfasst eine Mehrzahl von Fenstern 40, die wiederum entweder in Form von durchgehenden Ausnehmungen oder transparenten Bereichen ausgebildet sind. Entlang jedes Fensters 40 ist eine Ablesewertskala 42 angeordnet, deren Strichteilung sich entlang der jeweiligen Längserstreckung des Fensters 40, also in der Verschieberichtung 14 der ersten Schieberzunge 8, erstreckt. Auch die dritte Schieberzunge 38 umfasst ein Lochraster 44, welches in der Verschieberichtung 32 der dritten Schieberzunge 38 erstreckt ist.

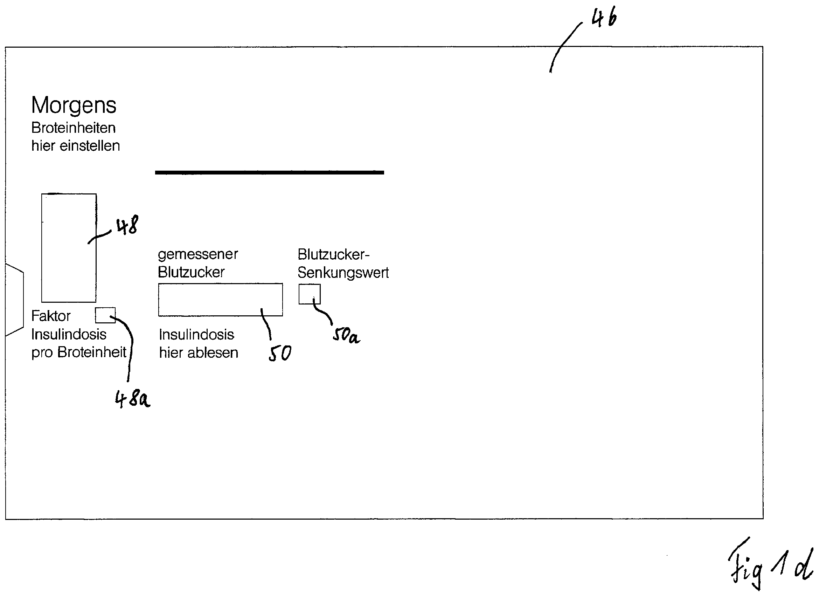

Figur 1d zeigt eine die Sichtseite bildende Deckschicht 46, in der Fenster 48 und 50 als durchgehende Ausnehmung oder als visuell transparenter Bereich eine visuelle Einsichtnahme auf vorstehend erwähnte Skalen der darunter befindlichen Schieberzungen gestatten. Durch dieselben Fenster 48 und 52 oder durch weitere Fenster 48a und 50a in der Deckschicht 46 sind erste Parameter 37 (Figur 1 c) und zweite Parameter 45 (Figur 1 c) der zweiten Schieberzunge 30 bzw. der dritten Schieberzunge 38 für den Benutzer visuell wahrnehmbar. Die Anzeige dieser ersten und zweiten Parameter vermittelt dem Benutzer eine dem Parameter entsprechende Positionierung oder Schiebestellung der zweiten Schieberzunge 30 und der dritten Schieberzunge 38.

Die eingangs erwähnte Verstellung der zweiten Schieberzunge 30 und der dritten Schieberzunge 38 geschieht nach der hier beispielhaft beschriebenen Ausführungsform wie folgt: Der Benutzer zieht die erste Schieberzunge 8 in Richtung des Pfeils 14 aus dem durch die feststehenden Schichten gebildeten Gehäusekörper 52 bis zu den Pfeilmarkierungen 54 (Figur 1b) heraus. In dieser Position fluchten die Fenster 20 und 22 in der ersten Schieberzunge 8 mit den Fenstern 4 und 6 in der rückseitigen Deckschicht 4 und gestatten somit einen Zugriff oder Durchgriff auf das Lochraster 36 der zweiten Schieberzunge 30 und auf das Lochraster 44 der dritten Schieberzunge 38. Der Benutzer kann dann mittels eines stiftförmigen an sich beliebigen Mittels durch das langlochförmige Fenster 4, 6 in der rückwärtigen Deckschicht 6 hindurch und durch die Fenster 20, 22 in das jeweilige Lochraster 36, 44 eingreifen und auf diese Weise die zweite und dritte Schieberzunge 30 bzw. 38 in der Richtung 32 inkrementell um eine vorgegebene Position verschieben. Dabei betrachtet der Benutzer den Gehäusekörper 52 der Schiebervorrichtung von hinten. Da die zweite und dritte Schieberzunge 30, 38 auf der Rückseite ebenfalls die ersten Parameter 37 und die zweiten Parameter 45 visuell wahrnehmbar aufweist, sind diese jeweils durch das rechteckförmige Fenster 4, 6 in der rückseitigen Deckschicht 2 sichtbar. Der Benutzer erhält so einen Hinweis auf den durch Verschiebung der jeweiligen Schieberzunge 30, 38 eingestellten Parameter. Nach Einstellung dieser Parameter, d. h. der intendierten Verschiebestellung der zweiten Schieberzunge 30 und der dritten Schieberzunge 38 wird die erste Schieberzunge 8 wieder eingeschoben. Somit gelangen die Fenster 20, 22 in der ersten Schieberzunge 8 aus dem Überlappungsbereich der Fenster 4 und 6 in der rückseitigen Deckschicht heraus. Ein Zugriff auf die zweite und dritte Schieberzunge 30, 38 ist nun nicht mehr möglich. Die zweite und dritte Schieberzunge 30, 38 sind auf diese Weise unverschiebbar gestellt, was ein unbeabsichtigtes Verstellen der Schieberzungen 30, 38 wirksam verhindert.

Bei einer alternativen nicht dargestellten Ausführungsform wäre es denkbar, dass ein Zugriff auf die zweite und dritte Schieberzungen, insbesondere auf die Lochraster 36, 44 der zweiten und dritten Schieberzunge 30, 38, über die sichtseitige Deckschicht 46 erfolgt, indem dort, insbesondere an entsprechender Stelle der Lochraster, vorzugsweise langgestreckte und vorzugsweise nur wenige Millimeter breite Fenster in Form von Durchgriffsausnehmungen vorgesehen sind.

Bei beiden Ausführungsformen wäre es möglich, dass die Fenster in der Deckschicht durch geeignete Mittel zur Zugriffsverhinderung verschließbar sind. Im einfachsten Fall könnte dort ein Klebeetikett oder ähnliches vorgesehen sein.

Für die Verwirklichung der Schiebervorrichtung zur Ermittlung einer zu spritzenden Insulinmenge sind die Eingabeskalen 16 entsprechend dem ersten Parameter 37 (Insulinfaktor) ausgebildet, d. h. in der vorstehenden Ausführungsform entspricht das Verhältnis der Strichteilung der Eingabeskalen zueinander dem Verhältnis der einer jeweiligen Eingabeskala zugeordneten ersten Parameter 37. Durch Einstellung der Schiebestellung der zweiten Schieberzunge wird die dem entsprechenden ersten Parameter 37 entsprechende Eingabeskala 16 in den Sichtbereich des Fensters 34 in der zweiten Schieberzunge 30 gebracht. Die Abstände der Strichteilung der jeweiligen Ablesewertskalen zueinander entsprechen bei dem hier gewählten Ausführungsbeispiel einer Division dem umgekehrten Verhältnis der einer jeweiligen Ablesewertskala 42 zugeordneten zweiten Parameter 45. Entsprechend der Schiebestellung der dritten Schieberzunge 38 wird das dem intendierten zweiten Parameter 45 entsprechende Fenster 40 der dritten Schieberzunge 38 in Überdeckung oder Überlappung mit der Ergebnisskala 18 auf der ersten Schieberzunge 8 gebracht. Für die Ermittlung der zu spritzenden Insulinmenge muss nun lediglich die erste Schieberzunge 8 derart weit herausgezogen werden, dass die Einstellmarke 35 im Bereich des Fensters 34 der zweiten Schieberzunge 30 auf die zugrunde zu legenden Brot- bzw. Kohlehydrateinheiten gestellt wird. Die zu spritzende Insulinmenge kann nun bei einem aktuell gemessenen Blutzuckerwert als Ablesemarkierung der Ablesewertskala 42 auf der darunter positionierten Ergebnisskala 18 der ersten Schieberzunge 8 abgelesen werden.

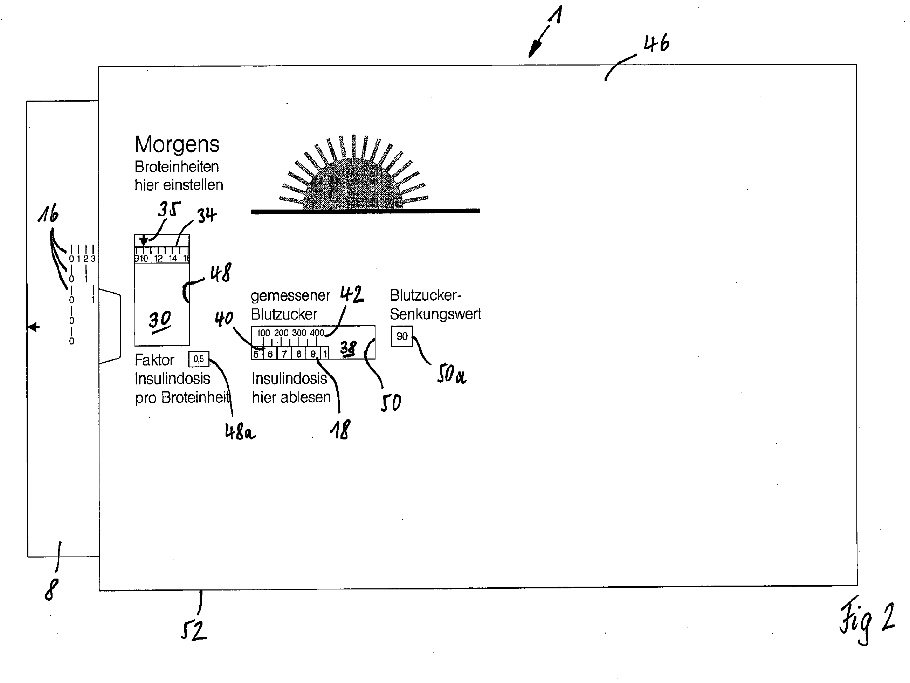

Figur 2 zeigt beispielhaft eine Betriebsposition der Schiebervorrichtung. Die zweite Schieberzunge 30 ist mit ihrem Fenster 34 in einer dem Insulinfaktor von 0,5 als erster Parameter entsprechenden Schiebestellung. Die dritte Schieberzunge 38 ist in einer dem Blutzucker-Senkungswert von 90 als zweiter Parameter entsprechenden Schiebestellung. Die erste Schieberzunge 8 ist derart weit herausgezogen, dass die Einstellmarke 35 auf den Brot- bzw. Kohlehydrateinheiten eingestellt ist. Wenn nun ein aktuell durch Messung ermittelter Blutzuckerwert des Patienten 200 beträgt, so kann die zu spritzende Insulinmenge auf der Ablesewertskala 42 direkt unterhalb des Werts "200" als Ablesemarke auf der Ergebnisskala 18 der ersten Schieberzunge 8 abgelesen werden. Im vorliegenden Fall ergibt sich ein Wert zwischen 6,5 und 7, also etwa 6,75 Insulineinheiten.

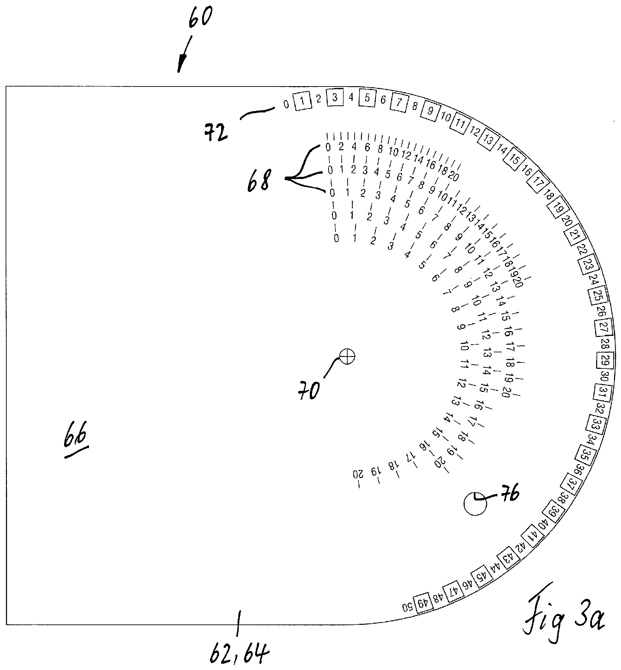

Figuren 3a bis 3e zeigen in verschiedenen Ebenen eine erfindungsgemäße Drehscheibenvorrichtung 60, wiederum in der Reihenfolge von unten nach oben, also in Richtung auf den Benutzer.

Figur 3a zeigt eine Draufsicht auf einen Flachmaterialabschnitt, der eine rückseitige Deckschicht 62 der Drehscheibenvorrichtung 60 bildet. Diese rückseitige Deckschicht bildet zugleich eine erste Drehscheibe 64. Sie ist jedoch nicht kreisscheibenförmig ausgebildet, sondern umfasst beispielhaft einen über die Projektion einer Kreisscheibe seitlich vorstehenden rechteckförmigen Abschnitt 66, an dem die Drehscheibenvorrichtung 60 bei der Bedienung gut ergriffen werden kann. Die erste Drehscheibe 64 umfasst des Weiteren mehrere Eingabeskalen 68, die in verschiedenen radialen Abständen und jeweils konzentrisch zu einer Drehachse 70 angeordnet oder erstreckt sind. Jeder Eingabeskala 68 ist dabei ein erster Parameter, nämlich der sogenannte Insulinfaktor, zugeordnet. Dies bedeutet, dass im beispielhaft dargestellten Fall, dass das Verhältnis der Abstände der Strichteilung der jeweiligen Eingabeskala 68 dem Verhältnis der einer jeweiligen Eingabeskala zugeordneten ersten Parameter entspricht. Des Weiteren ist auf der ersten Drehscheibe 64 beispielhaft radial außen eine Ergebnisskala 72 ebenfalls konzentrisch erstreckt vorgesehen.

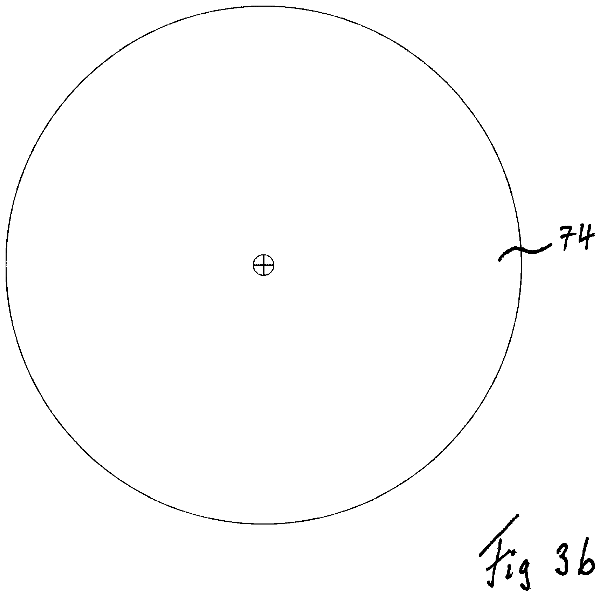

Figur 3b zeigt die Draufsicht auf eine optionale Abstandhalterscheibe 74, deren Radius kleiner ist als der Abstand einer noch zu erläuternden Durchgriffsausnehmung 76 in von der Drehachse 70. Diese Durchgriffsausnehmung 76 ist wenigstens in den nachfolgend zu beschreibenden Drehscheiben vorgesehen und optional auch in der ersten Drehscheibe 64.

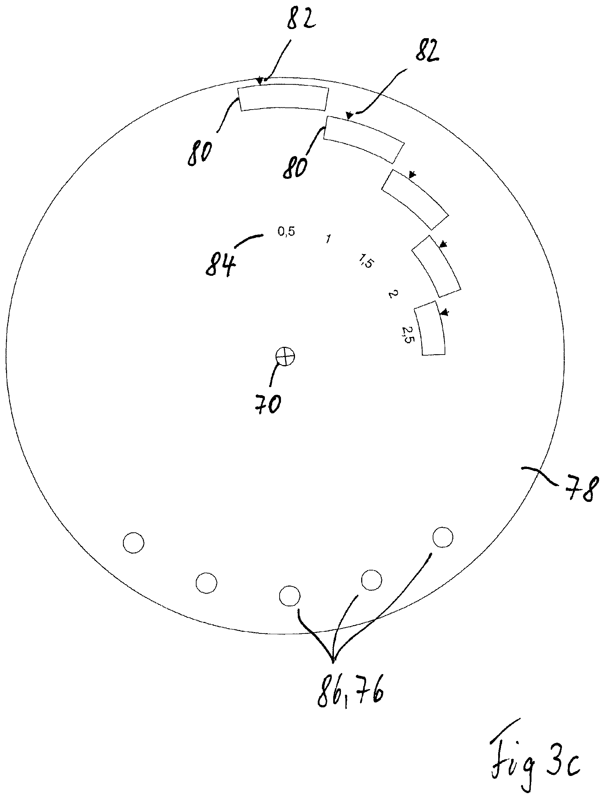

Figur 3c zeigt eine dritte Ebene der erfindungsgemäßen Drehscheibenvorrichtung 60. Sie umfasst eine zweite Drehscheibe 78 mit einer Mehrzahl von Fenstern 80, die in verschiedenen radialen Abständen zur Drehachse 70 vorgesehen sind. Je nach Drehstellung der zweiten Drehscheibe 78 relativ zur ersten Drehscheibe 64 kann relativ zu einer "12 Uhr Position" der ersten Drehscheibe eine der Eingabeskalen 68 der ersten Drehscheibe 64 in Überlappung mit einem der Fenster 80 in der zweiten Drehscheibe 78 gebracht werden. Ferner ist an jedem Fenster 80 eine Einstellmarke 82 vorgesehen, welche den Zweck hat, dass sie gegenüber einem Wert der Eingabeskala 68 positioniert wird.

Radial innerhalb der jeweiligen Fenster 80 und konzentrisch ist ferner der zu einem jeweiligen Fenster 80 bzw. zu einer jeweiligen Eingabeskala 68 gehörige erste Parameter 84, der vorerwähnte Insulinfaktor, visuell wahrnehmbar vorgesehen. Ferner weist die zweite Drehscheibe 78 ein konzentrisches Lochraster 86 auf, welches durch die schon erwähnten Durchgriffsausnehmungen 76 gebildet ist.

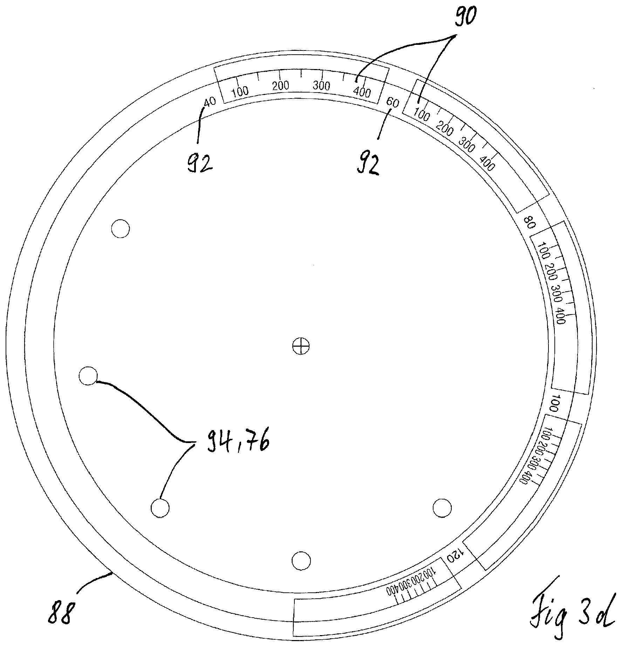

Figur 3d zeigt eine Draufsicht auf eine dritte Drehscheibe 88 mit einer Mehrzahl von konzentrisch und beispielhaft radial außen angeordneten Ablesewertskalen 90. Die Strichteilung dieser Ablesewertskalen 90 steht im beispielhaft dargestellten Fall in reziprokem Verhältnis zu einem einer jeweiligen Ablesewertskala 90 zugeordneten zweiten Parameter 92, dem Blutzucker-Senkungswert, der jeweils neben der betreffenden Ablesewertskala 90 ebenfalls visuell wahrnehmbar vorgesehen ist. Das reziproke Verhältnis ergibt sich aus einem eine Division beschreibenden Algorithmus. Die dritte Drehscheibe 88 ist radial innerhalb der Ablesewertskalen 90 durchsichtig ausgebildet. Auch sie weist ein Lochraster 94 aus Durchgriffsausnehmungen 76 auf, welches im selben radialen Abstand zur Drehachse 70 ausgebildet ist und im selben Winkelabstand wie das Lochraster 86 in der zweiten Drehscheibe 78. Die Reihenfolge der Anordnung der zweiten Drehscheibe 78 und der dritten Drehscheibe 88 kann auch umgekehrt sein und ist hier rein beispielhaft.

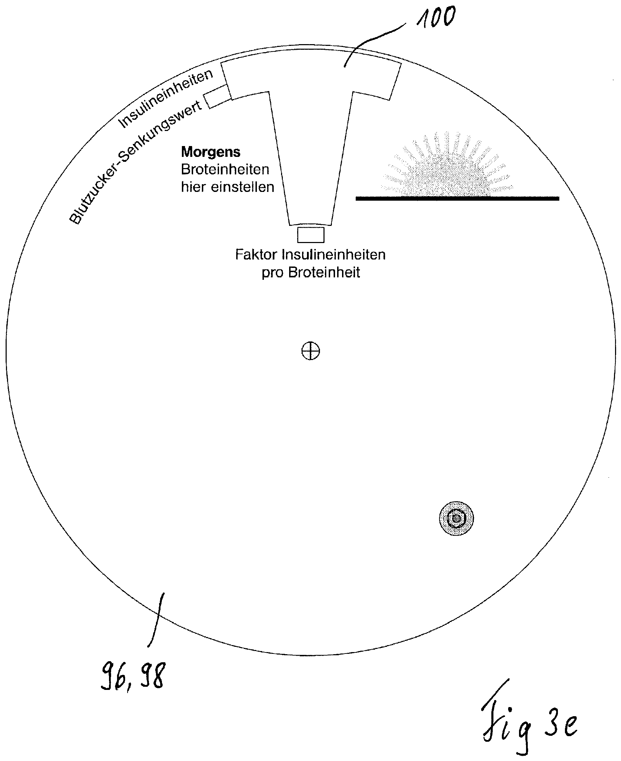

Schließlich zeigt Figur 3e einen beispielhaft kreisscheibenförmigen Flachmaterialabschnitt, der eine dem Betrachter zugewandte sichtseitige Deckschicht 96 und damit eine vierte Drehscheibe 98 bildet. Die vierte Drehscheibe 98 umfasst ein radial und in Umfangsrichtung erstrecktes Fenster 100, welches derart bemessen ist, dass es in einer wählbaren Drehstellung eine Einsichtnahme auf die in dieser Drehstellung einsehbaren Skalen oder Skalenbereiche der anderen Drehscheiben ermöglicht. Das Fenster 10 kann als durchgehende Ausnehmung oder als transparenter Bereich der vierten Drehscheibe 98 gebildet sein.

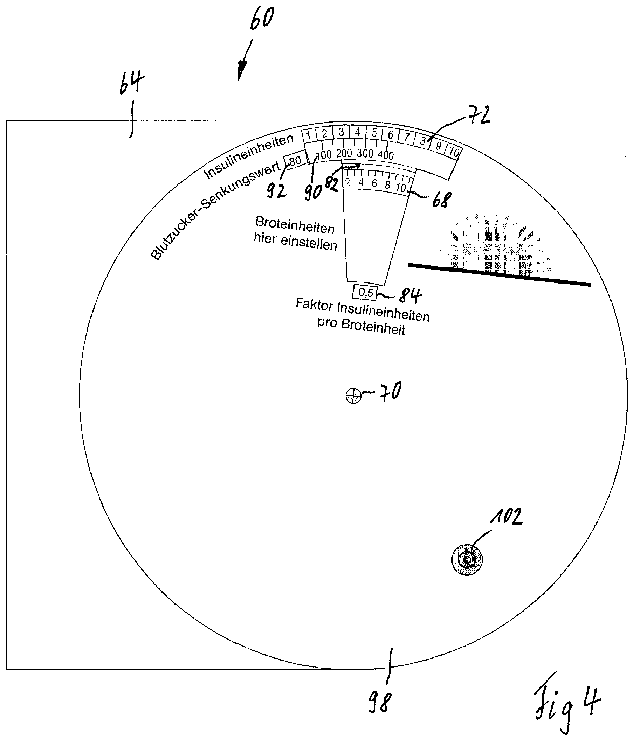

Anhand von Figur 4 ist eine Draufsicht auf die erfindungsgemäße Drehscheibenvorrichtung 60 unter Zugrundelegung der Parameter der oben erörterten Tabelle dargestellt. Dabei wurde die dem ersten Parameter 84 (Insulinfaktor) von 0,5 entsprechende Eingabeskala 68 ausgewählt. Ferner wurde die dem zweiten Parameter 92 (Blutzucker-Senkungswert) von 80 zugeordnete Ablesewertskala 90 zugrunde gelegt. Fixiert man nun die zweite Drehscheibe 78, die dritte Drehscheibe 88 und die vierte Drehscheibe 98 in der so orientierten Drehstellung zueinander, was beispielsweise durch ein splint-, schrauben- oder nietförmiges Element 102 erfolgen kann, welches in der betreffenden Drehstellung durch die miteinander axial fluchtenden Durchgriffsausnehmungen 76 des jeweiligen Lochrasters 86, 94 geschehen kann, so lässt sich der so fixierte Verbund gegenüber der ersten Drehscheibe 64 verdrehen. Zur Ermittlung einer zu spritzenden Insulinmenge wird nun eine solche Verdrehung dieses Verbunds gegenüber der ersten Drehscheibe 64 vorgenommen, dass die Einstellmarke 82 am betreffenden Fenster 80 der zweiten Drehscheibe 78 gegenüber den zu konsumierenden Brot- bzw. Kohlehydrateinheiten positioniert wird, im vorliegenden Fall also bei 3,5 Brot- bzw. Kohlehydrateinheiten. Wenn nun wie im Beispiel der Tabelle ein aktueller Blutzuckerwert von 120 gemessen wird, so braucht die zu spritzende Insulinmenge lediglich bei diesem Wert 120 der Ablesewertskala 90 als Ablesemarke radial außerhalb auf der Ergebnisskala 72 abgelesen zu werden. Er liegt in diesem Fall genau bei zwei Insulineinheiten.

Mit der Schiebervorrichtung 1 und der erfindungsgemäßen Drehscheibenvorrichtung 60 wurden somit bei zuvor eingestelltem erstem und zweitem Parameter durch eine einzige Schiebe- bzw. Drehstellbewegung eine Multiplikation und Division und Addition entsprechend vorgegebener Algorithmen ausgeführt. Dadurch, dass auch bei der Drehscheibenvorrichtung 60 die zweite, dritte und vorzugsweise auch die vierte Drehscheibe 78, 88, 98 gegeneinander unverdrehbar gestellt werden können, und zwar durch an sich beliebige Mittel, ist ein hohes Maß an Bediensicherheit realisiert. Auch hier können zusätzlich zur Überdeckung des splint-, schrauben- oder nietförmigen Elements 102 Klebeetiketten oder dergleichen eingesetzt werden.

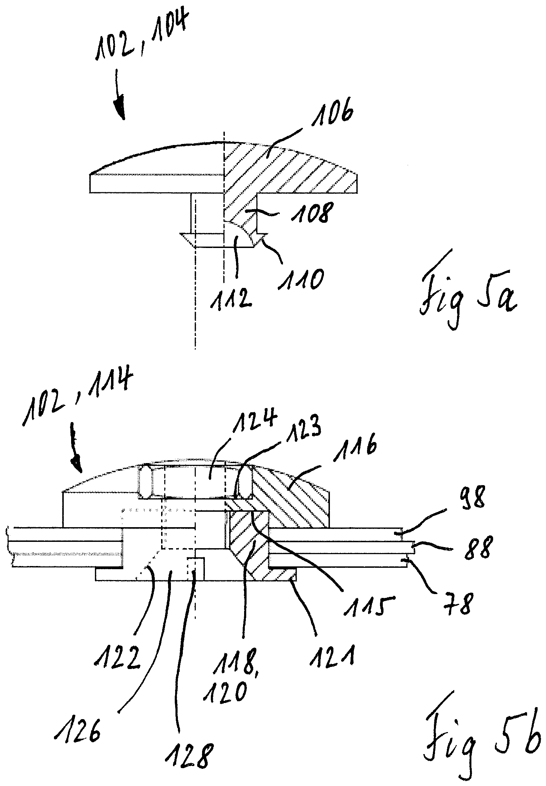

Figuren 5a und b zeigen zwei Ausführungsbeispiele für solche prinzipiell in Frage kommenden Elemente. Figur 5a zeigt einen Arretierungsknopf 104 mit einem flach gehaltenen, sich radial nach außen verjüngenden Kopf 106 und mit einem in axialer Richtung vorstehenden Stift oder Stamm 108. Am axialen freien Ende des Stifts oder Stamms 108 ist eine Widerhaken bildende radiale Erweiterung 110 ausgebildet, die sich aber in Einsteckrichtung wieder konisch verjüngt. Auf diese Weise kann der Arretierungsknopf 104 durch die zweite, dritte und vierte Drehscheibe 78, 88, 98 hindurchgesteckt werden, wobei die Bemessung der Durchgriffsausnehmungen 76 und des Stifts oder Stamms 108 einschließlich seiner radialen Erweiterung 110 derart ist, dass beim Hindurchstecken die radiale Erweiterung 110 mit ihrer konischen Ausbildung gegen die Durchgriffsausnehmungen 76 anläuft und elastisch nach radial innen verdrängt wird und sodann wieder nach radial außen schnappt und somit formschlüssig widerhakenartig verrastet. Dies wird durch eine in Figur 5a angedeutete axiale Ausnehmung 112 begünstigt. Bei diesem Ausführungsbeispiel des Elements 102 ist ein hohes Maß an Sicherheit gegen unbeabsichtigtes oder unwissentliches Verändern der intendierten Drehstellung der Scheiben zueinander realisiert.

Figur 5b zeigt eine weitere Ausführungsform eines Elements 102 in Form eines mehrteiligen Arretierungsknopfs 114. Er umfasst wiederum einen abgeflachten Kopf 116, einen Stift oder Stamm 118, jedoch in Form einer vom Kopf 116 lösbaren zylindrischen Hülse 120 mit Flansch 121 und mit konischer Innenansenkung 122, die in eine axiale Öffnung 123 am Kopf 116 eingesetzt ist. Ferner sind von beiden Seiten gegeneinander verschraubbare Schraubenmittel 124 und 126 eingesetzt, wobei das Schraubenmittel 124 in eine abgesetzte Bohrung im Kopf 116 und das Schraubenmittel 126 in die zylindrische Hülse 120 eingesetzt ist. Das eine Schraubenmittel 126 weist vorteilhafterweise eine axiale Werkzeugansetzstelle 128 auf. Diese Werkzeugansetzstelle 128 ist beispielsweise durch die optionale Durchgriffsausnehmung 76 in der ersten Drehscheibe 64 hindurch zugänglich, um die Schraubverbindung zu lösen bzw. herzustellen, wenn gewolltermaßen eine Veränderung der Drehstellung der Drehscheiben 78, 88, 98 herbeigeführt werden soll. Diese Durchgriffsausnehmung 76 kann erwünschtenfalls durch ein nicht zerstörungsfrei ablösbares Etikett verdeckt und damit gesichert werden.

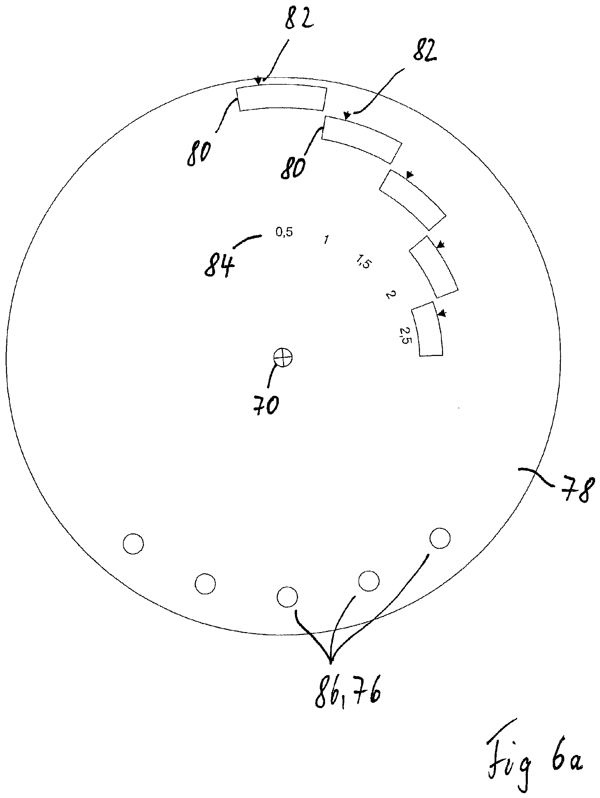

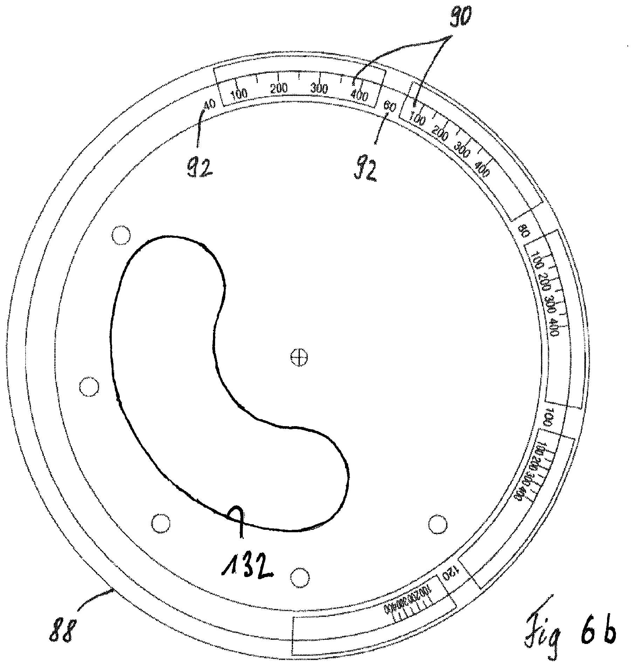

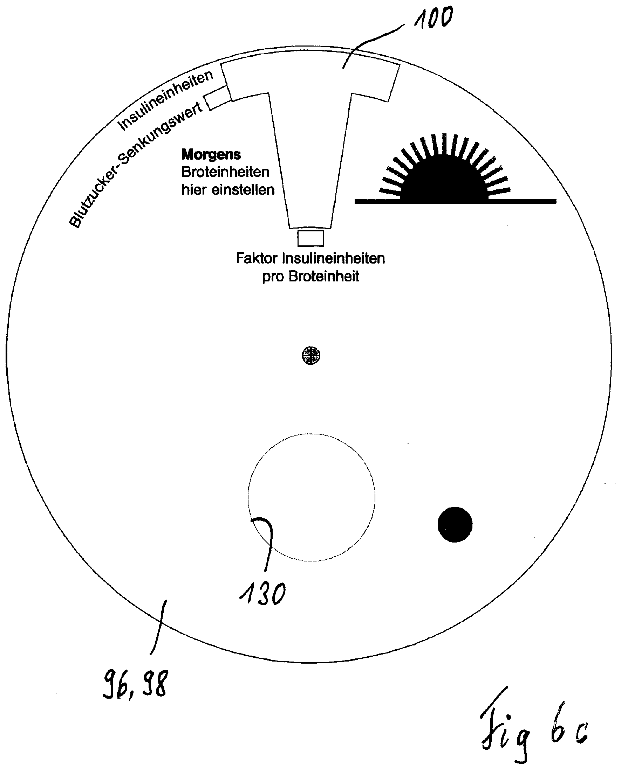

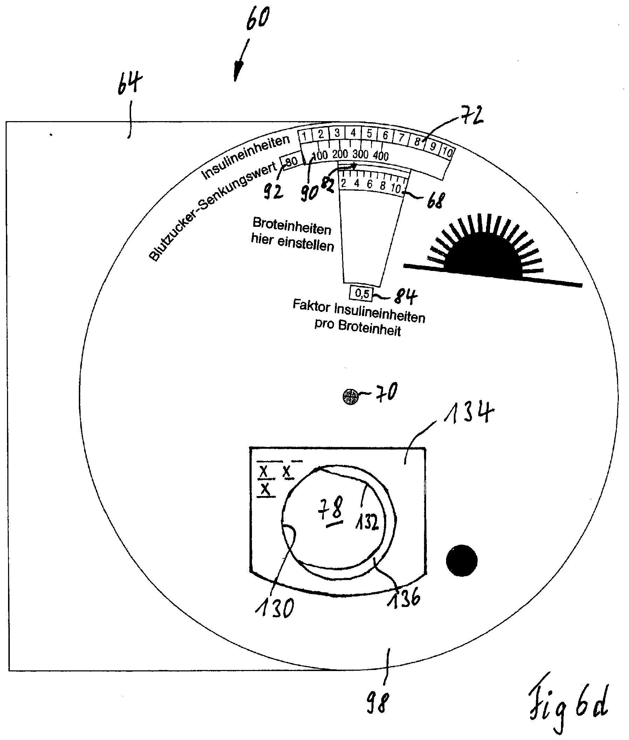

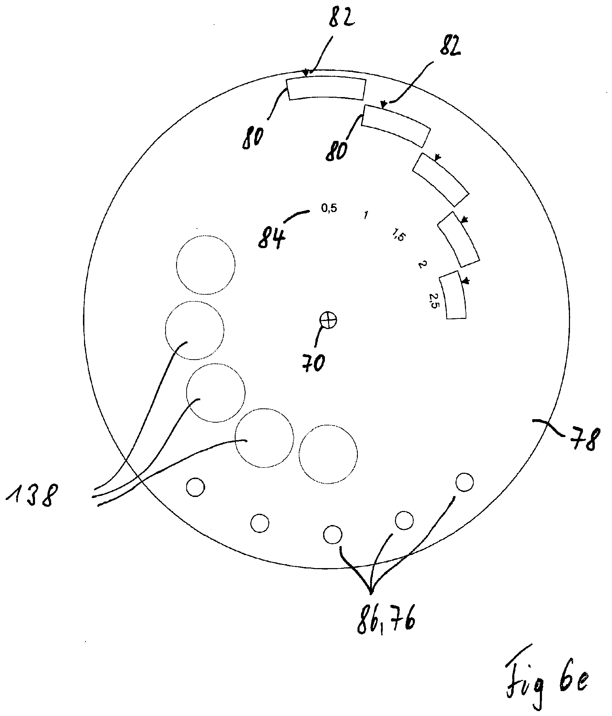

Die Figuren 6a - d zeigen eine weitere bevorzugte Möglichkeit zum Unverdrehbarstellen der zweiten, dritten und vierten Drehscheibe 78, 88, 98. Die Figuren 6a - c zeigen eine Draufsicht auf diese Drehscheiben 78, 88, 98. Man erkennt in der vierten, die Deckseite bildenden Drehscheibe 98 eine beispielhaft kreisrunde Ausnehmung 130 und in der darunter angeordneten dritten Drehscheibe 88 eine langgestreckte, beispielhaft nierenförmige oder langlochförmige Ausnehmung 132, die so angeordnet sind, dass sie miteinander bei entsprechender Drehstellung überlappen. Dies ist in Figur 6d angedeutet. Die Abmessung der Ausnehmung 132 in der dritten Drehscheibe 88 ist dabei zumindest in einer Richtung geringer als die Abmessung der Ausnehmung 130 in der vierten Drehscheibe 98. Durch die Ausnehmung 132 in der dritten Drehscheibe 88 hindurch ist ein Oberflächenbereich der zweiten Drehscheibe 78 exponiert. Bei umgekehrter Anordnungsreihenfolge der zweiten Drehscheibe 78 und der dritten Drehscheibe 88 wäre die langlochförmige Ausnehmung 132 in der zweiten Drehscheibe 88 auszubilden. Wie in Figur 6d dargestellt, können die drei Drehscheiben 78, 88 und 98 durch Aufbringen eines einzigen Klebeetiketts 134 miteinander fixiert, das heißt, gegeneinander unverdrehbar gestellt werden. Man erkennt aus Figur 6d, dass das Klebeetikett 134 auf dem die Öffnung 130 in der vierten Drehscheibe umgebenden Bereich sowie auf einem die Ausnehmung 132 umgebenden Bereich 136 der darunter befindlichen dritten Drehscheibe 88 sowie auf der Oberseite der zweiten Drehscheibe 78 klebend fixiert ist oder fixierbar ist. Das Klebeetikett 134 kann bei Ablösung selbstzerstörend ausgebildet sein, so dass hierdurch sichergestellt wird, dass die Drehscheibenanordnung vom zuständigen Arzt oder Therapeuten korrekt eingestellt und fixiert wurde. Das Klebeetikett kann außerdem als Informationsträger für Anmerkungen des behandelnden Arztes oder Therapeuten eingesetzt werden, insbesondere kann die gewählte Drehstellung auf der Sichtseite dokumentiert werden. Bei dieser Ausbildung ist die Vorsehung von Durchgriffsausnehmungen 76 für ein mechanisches Kopplungselement an sich nicht erforderlich oder lediglich als alternative Art der Fixierung vorgesehen. Schließlich zeigt Figur 6e eine weitere Ausführungsform mit mehreren Ausnehmungen 138 anstelle der einen langestreckten nierenförmigen Ausnehmung 132.

## Patentansprüche

1. Drehscheibenvorrichtung (60) zur Ermittlung einer zu spritzenden Insulinmenge, aus geschichtet angeordneten Flachmaterialabschnitten, die einen scheibenförmigen Gehäusekörper bilden, mit mehreren Drehscheiben, die um eine gemeinsame Drehachse (70), die orthogonal zu den Flachmaterialabschnitten verläuft, zueinander drehbar sind,
wobei eine erste Drehscheibe (64) mehrere Eingabeskalen (68) mit jeweils äquidistanter Strichteilung und eine Ergebnisskala (72) aufweist, wobei die mehreren Eingabeskalen (68) in verschiedenen radialen Abständen und jeweils konzentrisch zur Drehachse (70) angeordnet sind, wobei jeder Eingabeskala (68) ein erster Parameter (84) zugeordnet ist,
wobei eine zweite Drehscheibe (78) eine Mehrzahl von Fenstern (80) aufweist, die derart ausgebildet und in verschiedenen radialen Abständen und jeweils konzentrisch zur Drehachse (70) angeordnet sind, dass ein jeweiliges Fenster (80) bei entsprechend wählbarer Drehstellung der zweiten Drehscheibe (78) relativ zur ersten Drehscheibe (64) eine visuelle Einsichtnahme auf die Eingabeskala (68) ermöglicht, und ein jeweiliges Fenster (80) eine Einstellmarke (82) aufweist,
wobei eine dritte Drehscheibe (88) eine Mehrzahl von konzentrisch und in Umfangsrichtung aufeinanderfolgend angeordnete Ablesewertskalen (90) mit jeweils äquidistanter Strichteilung aufweist, wobei jeder Ablesewertskala (90) ein zweiter Parameter (92) zugeordnet ist, wobei die jeweilige Ablesewertskala (90) von radial innerhalb oder radial außerhalb an die Ergebnisskala (72) angrenzt,
wobei eine vierte Drehscheibe (98) als Deckscheibe vorgesehen ist, die ein radial und in Umfangsrichtung erstrecktes Fenster (100) aufweist, welches in einer wählbaren Drehstellung eine Einsichtnahme auf die Ablesewertskala (90) der dritten Drehscheibe (88) und auf die Ergebnisskala (72) der ersten Drehscheibe (64) und durch ein Fenster (80) der zweiten Drehscheibe (78) hindurch auf die Eingabeskala (68) der ersten Drehscheibe (64) ermöglicht,
wobei die zweite Drehscheibe (78) und die dritte Drehscheibe (88) und die vierte Drehscheibe (98) in einer gewählten Drehstellung zueinander unverdrehbar stellbar sind,
wobei nach Einstellung der Drehstellung der zweiten und dritten Drehscheibe (78, 80) relativ zueinander entsprechend dem Wert des ersten und zweiten Parameters (84, 92) die erste Drehscheibe (64) gegenüber der Einstellmarke (82) am Fenster (80) der zweiten Drehscheibe (78) positionierbar ist und dann bei einem Wert der Ablesewertskala (90) als Ablesemarke ein Wert auf der Ergebnisskala (72) ablesbar ist, der der zu spritzenden Insulinmenge entspricht.

2. Drehscheibenvorrichtung nach Anspruch 1, **dadurch gekennzeichnet, dass** zum Unverdrehbarstellen der zweiten, dritten und vierten Drehscheibe (78, 88, 98) eine insbesondere lösbare Klebeverbindung oder ein die Drehscheiben klemmschlüssig oder formschlüssig miteinander koppelndes mechanisches Element (102), insbesondere in Form einer Nietverbindung oder Schraubverbindung oder Verplombung, vorgesehen ist.

3. Drehscheibenvorrichtung nach Anspruch 1 oder 2, **dadurch gekennzeichnet, dass** das die zweite, dritte und vierte Drehscheibe (78, 88, 98) formschlüssig miteinander koppelnde mechanische Element eine Plombe aus zwei ineinandergreifenden und durch Lochausstanzungen in der zweiten, dritten und vierten Drehscheibe (78, 88, 98) hindurchgreifenden Kunststoffspritzgussteilen ist, wobei die Plombe eine Sollbruchstelle aufweist, an der sie bei einer beabsichtigten Neueinstellung der drei Drehscheiben (78, 88, 98) aufgebrochen und durch eine neue Plombe ersetzt werden kann.

4. Drehscheibenvorrichtung nach Anspruch 1, 2 oder 3, **dadurch gekennzeichnet, dass** die erste Drehscheibe (64) über die übrigen Drehscheiben (78, 88) radial vorsteht und dort eine Haltezone zum Ergreifen und Halten der Vorrichtung während der Bedienung bildet.

5. Drehscheibenvorrichtung nach einem oder mehreren der vorstehenden Ansprüche , **dadurch gekennzeichnet, dass** die Parameterwerte (84,92) in einer Umfangsrichtung aufeinanderfolgend visuell wahrnehmbar vorgesehen sind und in einer gewählten Drehstellung der zweiten und der dritten Drehscheibe (78, 88) zueinander der erste und der zweite Parameterwert (84,92) auf der Sichtseite der Vorrichtung visuell wahrnehmbar sind.

6. Drehscheibenvorrichtung nach einem oder mehreren der vorstehenden Ansprüche , **dadurch gekennzeichnet, dass** zum Unverdrehbarstellen der zweiten, dritten und vierten Drehscheibe (78, 88, 98) ein, insbesondere nicht zerstörungsfrei ablösbares, Klebeetikett (134) vorgesehen ist, welches auf die Sichtseite der vierten Drehscheibe (98) aufbringbar und dabei durch eine Ausnehmung (130) in der vierten Drehscheibe (98) hindurch auf der dritten Drehscheibe (88) anhaftbar und durch eine Ausnehmung (132) in der dritten Drehscheibe (88) hindurch auf der zweiten Drehscheibe (78) anhaftbar ist, so dass die zweite, dritte und vierte Drehscheibe (78, 88, 98) unverdrehbar zueinander gestellt sind.

7. Drehscheibenvorrichtung nach einem oder mehreren der vorstehenden Ansprüche , **dadurch gekennzeichnet, dass** zum Unverdrehbarstellen der zweiten, dritten und vierten Drehscheibe (78, 88, 98) ein Klebeetikett (134) vorgesehen ist, welches auf die Sichtseite der vierten Drehscheibe (98) aufbringbar und dabei durch eine Ausnehmung in der vierten Drehscheibe (98) hindurch auf der zweiten Drehscheibe (78) anhaftbar und durch eine Ausnehmung in der zweiten Drehscheibe (78) hindurch auf der dritten Drehscheibe (88) anhaftbar ist, so dass die zweite, dritte und vierte Drehscheibe (78, 88, 98) unverdrehbar zueinander gestellt sind.

8. Drehscheibenvorrichtung nach Anspruch 6 oder 7, **dadurch gekennzeichnet, dass** die Ausnehmung (132) in der zweiten oder dritten Drehscheibe (78, 88) langlochförmig, nierenförmig oder in Form einer Reihe von in Umfangs- oder Drehrichtung aufeinanderfolgend angeordneten Ausnehmungen (138) ausgebildet ist, wobei die Abmessung der Ausnehmung (132) oder der Ausnehmungen (138) in der zweiten oder dritten Drehscheibe (88) kleiner ist als die Abmessung der Ausnehmung (130) in der vierten Drehscheibe (98), so dass das Klebeetikett (134) auf einem Umgebungsbereich der Ausnehmung (132) oder der Ausnehmungen (138) in der zweiten oder dritten Drehscheibe (88) anhaftbar ist.

## Claims

1. A dial device (60) for ascertaining an amount of insulin to be injected, comprising portions of flat material arranged in layers which form a disk-like housing body, having a plurality of dials, which are rotatable relative to one another about a common axis of rotation (70) that extends orthogonally to the portions of flat material,
wherein a first dial (64) has a plurality of input scales (68), each with equidistant distribution of scale lines, and a output scale (72), and the plurality of input scales (68) are arranged in various radial spacings and concentrically to the axis of rotation (70), and each input scale (68) is assigned a first parameter (84);
wherein a second dial (78) has a plurality of windows (80), which are embodied in such a way and arranged in various radial spacings concentrically to the axis of rotation (70), that each respective window (80), given a suitably selectable rotary position of the second dial (78) relative to the first dial (64), enables a visual inspection of the input scale (68), and each respective window (80) has a setting mark (82);
wherein a third dial (88) has a plurality of reading scales (90), arranged concentrically and in succession in the circumferential direction, each with equidistant scale lines, and each reading scale (90) is assigned a second parameter (92), and the respective reading scale (90) borders the output scale (72) either radially inward or radially outward;
wherein a fourth dial (98) is provided as a cover disk, which has a window (100) which extends radially and in the circumferential direction and which in a selectable rotary position enables an inspection of the reading scale (90) of the third dial (88) and of the output scale (72) of the first dial (64) and, through a window (80) in the second dial (78), of the input scale (68) of the first dial (64);
wherein the second dial (78) and the third dial (88) and the fourth dial (98) can be positioned nonrotatably in a selected rotary position relative to one another; and
wherein after the rotary position of the second and third dials (78, 80) has been adjusted relative to one another, in accordance with a value of the first and second parameters (84, 92), the first dial (64) can be positioned opposite with respect to the setting mark (82) at the window (80) of the second dial (78) and then, at a value of the reading scale (90) as a reading mark, a value on the output scale (72) that is equivalent to the amount of insulin to be injected can be read out.

2. The dial device of claim 1, **characterized in that** for positioning the second, third and fourth dials (78, 88, 98) nonrotatably, an adhesive connection, which in particular can be undone, or a mechanical element (102) for coupling the dials by clamping or in form-locking fashion to one another is provided, in particular in the form of a rivet connection or screw connection or a seal.

3. The dial device of claim 1 or 2, **characterized in that** the mechanical element coupling the second, third and fourth dials (78, 88, 98) in form-locking fashion to one another is a seal comprising two plastic injection-molded parts, which mesh with one another and reach through stamped perforations in the second, third and fourth dials (78, 88, 98), and the seal has a rated breaking point, at which, upon an intended resetting of the three dials (78, 88, 98) can be broken off and replaced with a new seal.

4. The dial device of claim 1, 2 or 3, **characterized in that** the first dial (64) protrudes radially past the other dials (78, 88) and there forms a holding zone for grasping and holding the device while it is being used.

5. The dial device of one or more of the preceding claims, **characterized in that** the parameter values (84, 92) are provided successively in the circumferential direction visually perceptibly and, in a selected rotary position of the second and third dials (78, 88) relative to one another, the first and second parameter values (84, 92) are visually perceptible on the viewing side of the device.

6. The dial device of one or more of the preceding claims, **characterized in that** for positioning the second, third and fourth dials (78, 88, 98) nonrotatably, an adhesive label (134) is provided which in particular cannot removed without being destroyed, which can be applied to the viewing side of the fourth dial (98) and, through a recess (130) in the fourth dial (98), can be made to adhere to the third dial (88) and, through a recess (132) in the third dial (88), can be made to adhere to the second dial (78), so that the second, third and fourth dials (78, 88, 98) are positioned nonrotatably relative to one another.

7. The dial device of one or more of the preceding claims, **characterized in that** for positioning the second, third and fourth dials (78, 88, 98) nonrotatably, an adhesive label (134) is provided, which can be applied to the viewing side of the fourth dial (98) and, through a recess in the fourth dial (98), can be made to adhere to the second dial (78) and, through a recess in the second dial (78), can be made to adhere to the third dial (88), so that the second, third and fourth dials (78, 88, 98) are positioned nonrotatably relative to one another.

8. The dial device of claim 6 or 7, **characterized in that** the recess (132) in the second or third dial (78, 88) is embodied in the form of an oblong slot or kidney or in the form of a row of circumferentially or rotationally successively arranged recesses (138), and the size of the recess (132) or of the recesses (138) in the second or third dial (88) is smaller than the size of the recess (130) in the fourth dial (98), so that the adhesive label (134) can be made to adhere to a surrounding region of the recess (132) or of the recesses (138) in the second or third dial (88).

## Revendications

1. Dispositif à disques tournants (60) destiné à déterminer une quantité d'insuline à injecter, constitué de portions en matériau plat superposées qui forment un corps de boîtier en forme de disque, comprenant une pluralité de disques tournants qui peuvent tourner les uns par rapport aux autres autour d'un axe de rotation (70) commun qui s'étend orthogonalement aux portions en matériau plat,
dans lequel un premier disque tournant (64) présente une pluralité d'échelles d'entrée (68) ayant chacune une graduation équidistante et une échelle de résultat (72), lesdites plusieurs échelles d'entrée (68) étant disposées à des intervalles radiaux différents et chacune de manière concentrique à l'axe de rotation (70), à chaque échelle d'entrée (68) étant associé un premier paramètre (84),
dans lequel un deuxième disque tournant (78) comprend une pluralité de fenêtres (80) qui sont réalisées et disposées à des intervalles radiaux différents et chacune de manière concentrique à l'axe de rotation (70) de telle manière qu'une fenêtre (80) respective permet de jeter un regard sur l'échelle d'entrée (68) lorsque le deuxième disque tournant (78) se trouve dans une position de rotation sélectionnable de façon correspondante par rapport au premier disque tournant (64), et une fenêtre (80) respective présente un repère de réglage (82),
dans lequel un troisième disque tournant (88) comprend une pluralité d'échelles de valeur de lecture (90) chacune à graduation équidistante qui sont disposées de manière concentrique et de manière successive dans la direction circonférentielle, à chaque échelle de valeur de lecture (90) étant associé un deuxième paramètre (92), l'échelle de valeur de lecture (90) respective étant contiguë à l'échelle de résultat (72) radialement de l'intérieur ou radialement de l'extérieur,
dans lequel un quatrième disque tournant (98) est prévu en tant que disque de couverture qui présente une fenêtre (100) s'étendant radialement et dans la direction circonférentielle qui, dans une position de rotation sélectionnable, permet de jeter un regard sur l'échelle de valeur de lecture (90) du troisième disque tournant (88) et sur l'échelle de résultat (72) du premier disque tournant (64) et à travers une fenêtre (80) du deuxième disque tournant (78) sur l'échelle d'entrée (68) du premier disque tournant (64),
dans lequel le deuxième disque tournant (78) et le troisième disque tournant (88) et le quatrième disque tournant (98), dans une position de rotation choisie, peuvent être agencés de manière à ne pas pouvoir être tournés les uns par rapport aux autres,
dans lequel, après avoir réglé la position de rotation des deuxième et troisième disques tournants (78, 80) l'un par rapport à l'autre selon la valeur des premier et deuxième paramètres (84, 92), le premier disque tournant (64) peut être positionné en regard du repère de réglage (82) sur la fenêtre (80) du deuxième disque tournant (78), et, puis, à une valeur de l'échelle de valeur de lecture (90) en tant que repère de lecture, une valeur peut être lue sur l'échelle de résultat (72) qui correspond à la quantité d'insuline à injecter.

2. Dispositif à disques tournants selon la revendication 1, **caractérisé par le fait que** pour agencer les deuxième, troisième et quatrième disques tournants (78, 88, 98) de manière à ne pas pouvoir être tournés les uns par rapport aux autres, on prévoit un assemblage collé en particulier amovible ou un élément mécanique (102) couplant entre eux par serrage ou à engagement positif les disques tournants, en particulier sous forme d'un assemblage rivé ou d'un assemblage vissé ou d'un plombage.

3. Dispositif à disques tournants selon la revendication 1 ou 2, **caractérisé par le fait que** ledit élément mécanique couplant entre eux à engagement positif les deuxième, troisième et quatrième disques tournants (78, 88, 98) est un plomb se composant de deux pièces en matière plastique moulées par injection qui s'engagent l'une dans l'autre et traversent des poinçonnages réalisés dans les deuxième, troisième et quatrième disques tournants (78, 88, 98), dans lequel le plomb présente un point destiné à la rupture sur lequel il peut être rompu lors d'un nouveau réglage envisagé des trois disques tournants (78, 88, 98) et peut être remplacé par un nouveau plomb.

4. Dispositif à disques tournants selon la revendication 1, 2 ou 3, **caractérisé par le fait que** le premier disque tournant (64) dépasse radialement les disques tournants (78, 88) restants et y forme une zone de maintien pour prendre et maintenir le dispositif lors du maniement.

5. Dispositif à disques tournants selon l'une ou plusieurs des revendications précédentes, **caractérisé par le fait que** les valeurs de paramètre (84, 92) sont prévues de manière à pouvoir être perçues visuellement successivement dans une direction circonférentielle et que, dans une position de rotation choisie des deuxième et troisième disques tournants (78, 88) l'un par rapport à l'autre, les première et deuxième valeurs de paramètre (84, 92) peuvent être perçues visuellement sur la face visible du dispositif.

6. Dispositif à disques tournants selon l'une ou plusieurs des revendications précédentes, **caractérisé par le fait que** pour agencer les deuxième, troisième et quatrième disques tournants (78, 88, 98) de manière à ne pas pouvoir être tournés les uns par rapport aux autres, on prévoit une étiquette adhésive (134) qui, en particulier, ne peut pas être détachée sans être détruite et qui peut être appliquée sur la face visible du quatrième disque tournant (98) tout en étant apte à adhérer sur le troisième disque tournant (88) à travers un évidement (130) ménagé dans le quatrième disque tournant (98) et à adhérer sur le deuxième disque tournant (78) à travers un évidement (132) ménagé dans le troisième disque tournant (88) de sorte que les deuxième, troisième et quatrième disques tournants (78, 88, 98) sont agencés de manière à ne pas pouvoir être tournés les uns par rapport aux autres.

7. Dispositif à disques tournants selon l'une ou plusieurs des revendications précédentes, **caractérisé par le fait que** pour agencer les deuxième, troisième et quatrième disques tournants (78, 88, 98) de manière à ne pas pouvoir être tournés les uns par rapport aux autres, on prévoit une étiquette adhésive (134) qui peut être appliquée sur la face visible du quatrième disque tournant (98) tout en étant apte à adhérer sur le deuxième disque tournant (78) à travers un évidement ménagé dans le quatrième disque tournant (98) et à adhérer sur le troisième disque tournant (88) à travers un évidement ménagé dans le deuxième disque tournant (78) de sorte que les deuxième, troisième et quatrième disques tournants (78, 88, 98) sont agencés de manière à ne pas pouvoir être tournés les uns par rapport aux autres.

8. Dispositif à disques tournants selon la revendication 6 ou 7, **caractérisé par le fait que** ledit évidement (132) ménagé dans le deuxième ou le troisième disque tournant (78, 88) est réalisé sous forme de trou oblong, en forme de rein ou sous forme d'une série d'évidements (138) se succédant dans la direction circonférentielle ou de rotation, dans lequel la dimension de l'évidement (132) ou des évidements (138) ménagés dans le deuxième ou le troisième disque tournant (88) est inférieure à la dimension de l'évidement (130) ménagé dans le quatrième disque tournant (98) de sorte que ladite étiquette adhésive (134) peut adhérer sur une zone environnante de l'évidement (132) ou des évidements (138) ménagés dans le deuxième ou le troisième disque tournant (88).
